# EUROPEAN PATENT SPECIFICATION

(11) **EP 4 343 328 B1**
(45) Date of publication and mention of the grant of the patent: **11.03.2026**
(21) Application number: 23461639.9
(22) Date of filing: 25.08.2023
(51) Int. Cl.: G01N 33/569

(54) **DIAGNOSTIC METHOD AND COMPOSITION FOR THE DETECTION OF MASTITIS CAUSED BY E. COLI, S. UBERIS, S. AGALACTIAE OR S. AUREUS**
DIAGNOSTISCHES VERFAHREN UND ZUSAMMENSETZUNG ZUM NACHWEIS VON MASTITIS VERURSACHT DURCH E. COLI, S. UBERIS, S. AGALACTIAE ODER S. AUREUS
PROCÉDÉ ET COMPOSITION DE DIAGNOSTIC POUR LA DÉTECTION DE LA MAMMITE PROVOQUÉE PAR E. COLI, S. UBERIS, S. AGALACTIAE OU S. AUREUS

(30) Priority: 25.08.2022 PL 44209122
(43) Date of publication of application: 27.03.2024
(73) Proprietor: Uniwersytet Jagiellonski, 31-007 Krakow (PL); Uniwersytet Medyczny w Lodzi, 90-419 Lodz (PL); Uniwersytet Lodzki, 90-136 Lodz (PL)
(72) Inventor: Dobrut, Anna, 31-345 Kraków (PL); Wójcik-Grzybek, Dagmara, 30-638 Kraków (PL); Brzychczy-Wloch, Monika, 31-864 Kraków (PL); Bekier, Adrian, 02-776 Warszawa (PL); Skibinski, Jakub, 92-644 Lódz (PL); Rudnicka, Karolina, 91-017 Lódz (PL); Plocinski, Przemyslaw, 92-761 Lódz (PL)
(74) Representative: Witek, Rafal

(56) References cited:
- WO-A1-02/075310
- US-B1- 8 445 215
- MIHKLEPP K. ET AL: "Design and production of antibodies for the detection of Streptococcus uberis", ENZYME AND MICROBIAL TECHNOLOGY, vol. 96, 1 January 2017 (2017-01-01), US, pages 135 - 142, XP093134628, ISSN: 0141-0229, DOI: 10.1016/j.enzmictec.2016.10.009
- KIKU YOSHIO ET AL: "Evaluation of a rapid coliform detection kit from clinical mastitis milk using colloidal gold nanoparticle-based immunochromatographic strips", vol. 83, no. 11, 15 September 2021 (2021-09-15), JP, pages 1628 - 1633, XP055941750, ISSN: 0916-7250, Retrieved from the Internet <URL:https://www.ncbi.nlm.nih.gov/pmc/articles/PMC8636885/pdf/jvms-83-1628.pdf> DOI: 10.1292/jvms.21-0185
- JURONEN DELIA ET AL: "Immunosensing system for rapid multiplex detection of mastitis-causing pathogens in milk", TALANTA, vol. 178, 2017, pages 949 - 954, XP085275371, ISSN: 0039-9140, DOI: 10.1016/J.TALANTA.2017.10.043
- GOULART D�BORA BRITO ET AL: "Escherichia coli Mastitis in Dairy Cattle: Etiology, Diagnosis, and Treatment Challenges", vol. 13, 7 July 2022 (2022-07-07), Lausanne, XP093134664, ISSN: 1664-302X, Retrieved from the Internet <URL:https://www.ncbi.nlm.nih.gov/pmc/articles/PMC9301288/pdf/fmicb-13-928346.pdf> DOI: 10.3389/fmicb.2022.928346
- DOBRUT ANNA ET AL: "Detection of immunoreactive proteins of Escherichia coli, Streptococcus uberis, and Streptococcus agalactiae isolated from cows with diagnosed mastitis", FRONTIERS IN CELLULAR INFECTION MICROBIOLOGY, vol. 13, 10 February 2023 (2023-02-10), CH, XP093134626, ISSN: 2235-2988, DOI: 10.3389/fcimb.2023.987842

## Description

The invention concerns a diagnostic method and composition for detecting mastitis caused by *E. coli, S. uberis, S. agalactiae* or *S. aureus.*

Bovine mastitis (inflammation of the udder), generating millions of dollars in losses for cattle farmers, is an important veterinary issue [1]. It is estimated that mastitis is confirmed in up to 20% of cows in countries considered leaders in milk production [2]. In addition, economic losses sustained due to the clinical type of mastitis may be as high as $76,000 per month in each farm [3]. The losses are caused first of all by the decreased volume of milk produced, its lower quality and they may also result from the need for isolating affected animals for the time of treatment whereby animals not only do not produce milk but also need to be treated [1].

Bacteria are listed as the most common pathogens responsible for mastitis, such as: *Staphylococcus aureus, Streptococcus uberis, Streptococcus agalactiae, Streptococcus dysgalactiae, Corynebacterium bovis, Mycoplasma bovis, Escherichia coli, Klebsiella pneumoniae,* but also fungi and algae [4, 5].

Mastitis may be a subclinical, clinical or chronic infection. The type depends for example on the etiological agent, age, race, the animal's resistance and lactation status. The chronic type is the least frequent, but it results in the permanent inflammation of the mammary gland. The clinical form is the most dangerous, diagnosed based on visible and palpable symptoms, such as fever, edema, pain and redness of the mammary gland; in addition, the volume of milk produced decreases and changes in milk appearance develop. Furthermore, the subclinical form generates the largest losses in dairy industry because no evident symptoms are seen [6, 7, 8].

The evaluation of mastitis is currently based mainly on rapid tests that estimate the somatic cell count (SCC) in a test sample of cow's milk. This approach promptly provides diagnosis and, what is most important, the tests can be performed directly next to the animal. This is one of the most important advantages considering the type of field work of veterinarians. Nevertheless, the key limitation of the method is that the etiological agent cannot be identified. Because nothing is known about the pathogen responsible for the infection, targeted antibiotic therapy cannot be started, with a potential consequence of an increasing percentage of multi-resistant bacterial strains, a growing problem worldwide.

The widely used methods to identify the etiological agent, that is, culture-based, molecular, serology or spectrometric techniques (such as MALDI-ToF), are more time-consuming, require specialized equipment and qualified staff and unambiguous interpretation of the results is frequently difficult. However, the most important aspect is that they cannot be completed outside a laboratory, which significantly limits their usefulness in the diagnosis of bovine diseases.

Patent application US2003073073A1 concerns a multiplex immunochromatographic test (lateral flow assay, LFA) for the diagnosis of mastitis both in cows and in other dairy animals. For example, a protein known as fibronectin binding protein was claimed for *Staphylococcus aureus,* whose variant A is claimed in this application. The same proteins are not included for other species (*E. coli, S. agalactiae, S. uberis*).

Patent application WO2019216775A1 also discloses an LFA for the multiplex diagnosis of mastitis, but other antigens are targeted.

Patent application US2005260695A1 concerns the immunodiagnostics of mastitis based on lactoferrin, for example.

Patent US9556281B2 concerns protein A (SpA) for *Staphylococcus aureus* as an antigen used in the treatment and prevention of infections caused by the bacteria.

Patent application CA2270404A1 concerns a diagnostic marker for *Streptococcus uberis* known as lactoferrin binding protein, which is not included in the pool of proteins included in the application.

In addition, patent application EP1532253A1 describes a component of a vaccine against *Streptococcus uberis* in the form of proteins with a mass of approx. 22 kDa.

Patent application WO2075310A1 discloses a method for the detection of multiple pathogens in the milk of animals with suspected mastitis. The method uses pathogen-specific antibodies as detection tools.

The objective of the invention is to provide a diagnostic test easy to perform also in field conditions which would simultaneously fulfill the following requirements:
- it will ensure simultaneous identification of four bacterial species, that is *Staphylococcus aureus, Streptococcus agalactiae, Streptococcus uberis* and *Escherichia coli,* one of the most commonly isolated species responsible for mastitis,
- the duration of the test and waiting for results will not exceed 20 minutes,
- the test will ensure high sensitivity, specificity and/or accuracy of at least 75%,
- the test could be performed in field conditions directly next to the animal by a person without qualifications in veterinary diagnostics/medicine (e.g., the breeder).

The hereinabove objective has surprisingly been achieved owing to the present invention.

The object of the invention is a method for detecting mastitis caused by *E. coli, S. uberis, S. agalactiae* or *S. aureus,* characterized in that the following antigens are tested in a milk collected from the udder of a tested cow:
- ompA protein from *E. coli* having amino acid sequence SEQ ID NO. 1: 
MKKTAIAIAVALAGFATVAQAAPKDNTWYTGAKLGWSQYHDTGFINNNGPTHENQ
LGAGAFGGYQVNPYVGFEMGYDWLGRMPYKGSVENGAYKAQGVQLTAKLGYPIT
DDLDIYTRLGGMVWRADTKSNVYGKNHDTGVSPVFAGGVEYAITPEIATRLEYQWT
NNIGDAHTIGTRPDNGMLSLGVSYRFGQGEAAPVVAPAPAPAPEVQTKHFTLKSDVL
FNFNKATLKPEGQAALDQLYSQLSNLDPKDGSVVVLGYTDRIGSDAYNQGLSERRA
QSVVDYLISKGIPADKISARGMGESNPVTGNTCDNVKQRAALIDCLAPDRRVEIEVKG
IKDVVTQPQA
 or epitopes contained therein, listed in Table 1 having an amino acid sequence selected from SEQ ID NO. 5-13,
- EF-Tu protein from *S. uberis* having amino acid sequence SEQ ID NO. 2: 
MAKEKYDRSKPHVNIGTIGHVDHGKTTLTAAITTVLARRLPTSVNQPKDYASIDAAP
EERERGITINTAHVEYETETRHYAHIDAPGHADYVKNMITGAAQMDGAILVVASTDG
PMPQTREHILLSRQVGVKHLIVFMNKIDLVDDEELLELVEMEIRDLLSEYDFPGDDLP
VIQGSALKALEGDSKYEDIIMELMKTADEYIPEPERDTDKPLLLPVEDVFSITGRGTVA
SGRIDRGTVRVNDEIEIVGIKEETKKAVVTGVEMFRKQLDEGLAGDNVGILLRGVQR
 
DEIERGQVIAKPGSINPHTKFKGEVYILSKDEGGRHTPFFNNYRPQFYFRTTDVTGSIE
LPAGTEMVMPGDNVTISVELIHPIAVEQGTTFSIREGGRTVGSGIVSEIEA
 or epitopes contained therein, listed in Table 1 having an amino acid sequence selected from SEQ ID NO. 14-24,
- ADA protein from *S. agalactiae* having amino acid sequence SEQ ID NO. 3: 
MTQAVLKELAKAELHCHLDGSLSLPAIRKLANMADIILPSSDKELRKYVIAPAQTESL
VDYLKTFEFIRPLLQTKEALRFAAYDVARQAALENVIYIEIRFAPELSMDKGLTASDT
VLAVLEGLADAQKEFNIVARALVCGMRQSSHKTTKDIIKHIVDLAPKGLVGFDFAGD
EFSYPTDSLVDLIQEVKRSGYPMTLHAGECGCAKHIADSLNLGIKRMGHVTALTGQR
DLIKRFVEEDVVAEMCLTSNLQTKAASSIQSFPYQELYDAGGKITINTDNRTVSDTNL
TKEYSLFVTYFGTKIEDFLVFNQNAVKASFTSDSEKDTLLHKLQENYDSYLK
 or epitopes contained therein, listed in Table 1 having an amino acid sequence selected from SEQ ID NO. 25-32, and
- FnBPA protein from *S. aureus* having amino acid sequence SEQ ID NO. 4, 
MKNSLRYGIRKHKLGAASVFLGTMIVVGMGQDKEAAASEQNTTVEENGNSATENKS
KQTTTNNVNNIDETQTYSATSTEQPSNATQVTAEAAPQAVEAPQTSQAPQKVQSTNV
ETVKEEVVKEEVQPQVKETARSQENSGDQRQVDLTPKKVTQNQGTEPQVEVAQPRT
VSESKPRVTRSTDVAEAKDASEIKGTDVTSKVTVESGSIEAPQGNKVEPHAGQRVVL
KYKLKFEKGLHKDDYFDFTLSNNVNTHGVSTARKVPDIKNGTVVMATGQLLGNGKI
RYTFTDYIDYKVNVTADLEINLFIDPKTVQSNGQQIITSTLNDKETRTTLPIQYNPGVS
NSYANVNGSIETFDKGNNKFTHVAYIKPQNGHKSDSVSITGTLTQGSKANGNAPTVK
VYEVLKDAKELPESVYANISDSTMFKDVTQEMKDKLKVENNGSYKLDIEKLEKSYVI
HYDGEYLSGSDQVNFRTHMFGYPEQQYKYYYTHLGYQLTWDNGLVLYSNKAKGD
GTNGTITESNNMTFDEEYGNGVITGQYDKNLVTTVEEEYDSSTLDIDYHTAIDGEGG
YVDGYIETIEETDSSAIDIDYHTAVDSEAGHVGGYTESSEESNPIDFEESTHENSKHHA
DVVEYEEDTNPGGGQVTTESNLLEFDEESTKGIVTGAISDHTTVEDTKEYTTESNLIEL
VDELPEEHGQAQGPIEEITENNQHISHSGLGTENGHGNYGIVEEIEENSHVDIKSELGY
EGGQNIGNQSFEEDTEEDKPKYEQGGNIVDIDFDSVPQIQGQNKGDQSFEEDTEEDKP
KYEQGGNIVDIDFDSVPQIQGQNKGNQSFEEDTEKDKPKYEHGGNIIDIDFDSVPHIHG
FNKHTEIIEEDTNKDKPSYQFGGHNSVDFEEDTLPQVSGQNEGQQTIEEDTTPPIVPPT
PPTPEVPSEPETPTPPTPEVPSEPETPTPTPPTPEVPSEPETPTPPTPEVPSEPETPTPTPPTP
EVPSEPETPTPPTPEVPSEPETPTPPMPEIPAEPGKPVPPANEEPKKPSKPVEQGKVVTP
VIEINEKVKAVKPAKTSQSKKTELPETGGEETTNNGVLFGGLLSIIGLVLLRRNKKNH
KA
or epitopes contained therein, listed in Table 1 having an amino acid sequence selected from SEQ ID NO. 33-51,
wherein:
- confirmed presence of the antigen from *E. coli* indicates that the udder of the cow tested is infected by the species *E. coli* causing mastitis,
- confirmed presence of the antigen from *S. uberis* indicates that the udder of the cow tested is infected by the species *S. uberis* causing mastitis,
- confirmed presence of the antigen from *S. agalactiae* indicates that the udder of the cow tested is infected by the species *S. agalactiae* causing mastitis,
   and
- confirmed presence of the antigen from *S. aureus* indicates that the udder of the cow tested is infected by the species *S. aureus* causing mastitis,

Another object of the invention concerns a diagnostic composition for detecting mastitis caused by *E. coli, S. uberis, S. agalactiae* or *S. aureus,* characterized in that it contains antibodies useful for detecting the following antigens:
- ompA protein from *E. coli* having amino acid sequence SEQ ID NO. 1: 
MKKTAIAIAVALAGFATVAQAAPKDNTWYTGAKLGWSQYHDTGFINNNGPTHENQ
LGAGAFGGYQVNPYVGFEMGYDWLGRMPYKGSVENGAYKAQGVQLTAKLGYPIT
DDLDIYTRLGGMVWRADTKSNVYGKNHDTGVSPVFAGGVEYAITPEIATRLEYQWT
NNIGDAHTIGTRPDNGMLSLGVSYRFGQGEAAPVVAPAPAPAPEVQTKHFTLKSDVL
FNFNKATLKPEGQAALDQLYSQLSNLDPKDGSVVVLGYTDRIGSDAYNQGLSERRA
QSVVDYLISKGIPADKISARGMGESNPVTGNTCDNVKQRAALIDCLAPDRRVEIEVKG
IKDVVTQPQA
 or epitopes contained therein, listed in Table 1 having an amino acid sequence selected from SEQ ID NO. 5-13,
- EF-Tu protein from *S. uberis* having amino acid sequence SEQ ID NO. 2: 
MAKEKYDRSKPHVNIGTIGHVDHGKTTLTAAITTVLARRLPTSVNQPKDYASIDAAP
EERERGITINTAHVEYETETRHYAHIDAPGHADYVKNMITGAAQMDGAILVVASTDG
PMPQTREHILLSRQVGVKHLIVFMNKIDLVDDEELLELVEMEIRDLLSEYDFPGDDLP
VIQGSALKALEGDSKYEDIIMELMKTADEYIPEPERDTDKPLLLPVEDVFSITGRGTVA
SGRIDRGTVRVNDEIEIVGIKEETKKAVVTGVEMFRKQLDEGLAGDNVGILLRGVQR
DEIERGQVIAKPGSINPHTKFKGEVYILSKDEGGRHTPFFNNYRPQFYFRTTDVTGSIE
LPAGTEMVMPGDNVTISVELIHPIAVEQGTTFSIREGGRTVGSGIVSEIEA
 or epitopes contained therein, listed in Table 1 having an amino acid sequence selected from SEQ ID NO. 14-24,
- ADA protein from *S. agalactiae* having amino acid sequence SEQ ID NO. 3: 
MTQAVLKELAKAELHCHLDGSLSLPAIRKLANMADIILPSSDKELRKYVIAPAQTESL
VDYLKTFEFIRPLLQTKEALRFAAYDVARQAALENVIYIEIRFAPELSMDKGLTASDT
VLAVLEGLADAQKEFNIVARALVCGMRQSSHKTTKDIIKHIVDLAPKGLVGFDFAGD
EFSYPTDSLVDLIQEVKRSGYPMTLHAGECGCAKHIADSLNLGIKRMGHVTALTGQR
DLIKRFVEEDVVAEMCLTSNLQTKAASSIQSFPYQELYDAGGKITINTDNRTVSDTNL
TKEYSLFVTYFGTKIEDFLVFNQNAVKASFTSDSEKDTLLHKLQENYDSYLK
 or epitopes contained therein, listed in Table 1 having an amino acid sequence selected from SEQ ID NO. 25-32, and
- FnBPA protein from *S. aureus* having amino acid sequence SEQ ID NO. 4, 
MKNSLRYGIRKHKLGAASVFLGTMIVVGMGQDKEAAASEQNTTVEENGNSATENKS
KQTTTNNVNNIDETQTYSATSTEQPSNATQVTAEAAPQAVEAPQTSQAPQKVQSTNV
ETVKEEVVKEEVQPQVKETARSQENSGDQRQVDLTPKKVTQNQGTEPQVEVAQPRT
VSESKPRVTRSTDVAEAKDASEIKGTDVTSKVTVESGSIEAPQGNKVEPHAGQRVVL
KYKLKFEKGLHKDDYFDFTLSNNVNTHGVSTARKVPDIKNGTVVMATGQLLGNGKI
RYTFTDYIDYKVNVTADLEINLFIDPKTVQSNGQQIITSTLNDKETRTTLPIQYNPGVS
NSYANVNGSIETFDKGNNKFTHVAYIKPQNGHKSDSVSITGTLTQGSKANGNAPTVK
 
VYEVLKDAKELPESVYANISDSTMFKDVTQEMKDKLKVENNGSYKLDIEKLEKSYVI
HYDGEYLSGSDQVNFRTHMFGYPEQQYKYYYTHLGYQLTWDNGLVLYSNKAKGD
GTNGTITESNNMTFDEEYGNGVITGQYDKNLVTTVEEEYDSSTLDIDYHTAIDGEGG
YVDGYIETIEETDSSAIDIDYHTAVDSEAGHVGGYTESSEESNPIDFEESTHENSKHHA
DVVEYEEDTNPGGGQVTTESNLLEFDEESTKGIVTGAISDHTTVEDTKEYTTESNLIEL
VDELPEEHGQAQGPIEEITENNQHISHSGLGTENGHGNYGIVEEIEENSHVDIKSELGY
EGGQNIGNQSFEEDTEEDKPKYEQGGNIVDIDFDSVPQIQGQNKGDQSFEEDTEEDKP
KYEQGGNIVDIDFDSVPQIQGQNKGNQSFEEDTEKDKPKYEHGGNIIDIDFDSVPHIHG
FNKHTEIIEEDTNKDKPSYQFGGHNSVDFEEDTLPQVSGQNEGQQTIEEDTTPPIVPPT
PPTPEVPSEPETPTPPTPEVPSEPETPTPTPPTPEVPSEPETPTPPTPEVPSEPETPTPTPPTP
EVPSEPETPTPPTPEVPSEPETPTPPMPEIPAEPGKPVPPANEEPKKPSKPVEQGKVVTP
VIEINEKVKAVKPAKTSQSKKTELPETGGEETTNNGVLFGGLLSIIGLVLLRRNKKNH
KA
or epitopes contained therein, listed in Table 1 having an amino acid sequence selected from SEQ ID NO. 33-51,

Preferably, the diagnostic composition of the invention is characterized in that it contains monoclonal or polyclonal antibodies specific for the hereinabove antigens.

Preferably, the diagnostic composition of the invention contains labelled antibodies.

### Detailed description of the invention

One of the aspects of the invention is a multiplex immunodiagnostic test for detecting bacterial antigens representative for 4 bacterial species: *E. coli, S. uberis, S. agalactiae* and *S*. *aureus,* which are the most common etiological agents responsible for mastitis in cows.

According to the invention, a total of 14 immunogenic proteins were identified having antigenic potential defined as species specificity and immunoreactivity, and also associated with the bacterial cell surface. The properties could be confirmed both through bioinformatic analysis and experimental laboratory tests using SDS-PAGE and immunoblotting based on antibodies from animals infected by one of the four hereinabove bacteria and also from healthy cows (negative control, animals without mastitis). In addition, strains from animals with mastitis with etiologies other than *E. coli, S. uberis, S. agalactiae* or *S. aureus* were used in the experiments to rule out cross-reactivity.

In an example embodiment, proteins (ompA for *E. coli,* EF-Tu for *S. uberis,* ADA for *S. agalactiae,* FnBPA for *S. aureus*) selected according to the invention and monoclonal antibodies against these proteins were used to develop a rapid multiplex immunochromatographic test based on which bacteria found in milk from cows with mastitis can be detected.

In a preferable embodiment of the invention, the antigens identified by the present inventors are detected by monoclonal antibodies, the key component of the immunochromatographic test (lateral flow assay, LFA).

In an example embodiment of the invention discussed below, the said test is in the form of a cassette immunochromatographic test (lateral flow assay, LFA).

Surprisingly, the test based on the antigens specified according to the invention ensures high sensitivity, specificity and/or accuracy of at least 75%.

### Example 1. Identification of antigens useful for detecting mastitis caused by E. coli, S. uberis, S. agalactiae or S. aureus

### Identification of antigens specific for pathogenic Staphylococcus aureus, Streptococcus agalactiae, Streptococcus uberis and Escherichia coli species responsible for mastitis in cows

### Sample preparation

Twenty-two milk samples (50 mL) were collected from infected quarters of cow's udders in which clinical symptoms of mastitis were identified, such as edema, elevated temperature, hardening, redness and tenderness of the udder, changes in milk consistency (watery, pus or clots). The samples were collected within routine milk collection procedures by veterinarians from the "Omnivet" Veterinary Clinic in Orzesze and the "Egida" Veterinary Clinic in Wizna. In addition, 12 milk samples were collected from healthy animals, and constituted negative controls. Furthermore, a milk samples from a cow with infection caused by *Streptococcus dysgalactiae* was a cross-reactivity control between the tested isolates of the genus *Streptococcus.*

Nineteen blood samples (20 mL) were collected by single injection into the jugular vein within routine diagnostic tests to detect mastitis (positive samples). In addition, 12 blood specimens from healthy animals (negative controls) were collected by veterinarians within routine blood chemistry tests.

The milk and sera were stored at -80°C.

### Bacterial culture and identification

100 µL of each milk sample was cultured in the Columbia medium with sheep blood added (Difco) in aerobic conditions at 37°C for 24 hours. Subsequently, the samples preliminarily identified as *E. coli* were cultured in the McConkey medium (Oxoid) in aerobic conditions at 37°C for 24 hours to confirm the species. In addition, colonies showing morphology consistent with one of the four species sought were further analyzed to confirm the species. Identification included culture in the Granada Agar medium (bioMérieux) dedicated for the detection of β-hemolyzing *Streptococcus agalactiae* strains and the chromogenic CHROMagar^{™} Mastitis GP medium (CHROMagar^{™}) used to differentiate between the most common Gram-positive species causing mastitis in cows. Identification was also performed based on the CAMP test, catalase test, API test and MALDI-ToF technique.

### Protein isolation and detection

Bacterial strains were isolated based on a modified procedure of Park et al. [9], developed based on a protocol reported by Fang and Oliver [10]. Modifications included for example culture in liquid tryptic soy broth (TSB, Becton-Dickinson) at 37°C for 24 hours. After the end of culture, the bacterial suspension was centrifuged (3000 rpm for 30 min), and the supernatant was removed and the bacterial pellet was suspended in 3 mL phosphate buffered saline (PBS, Thermo Fisher) and centrifuged for 20 minutes (3300 rpm). The procedure was repeated three times for each sample and wet bacterial mass was determined; 100 µL of 0.2% sodium dodecyl sulfate (SDS, Sigma Aldrich) was added per each 30 mg of the wet cell mass, and the samples were incubated at 43°C for 1 hour. Subsequently, the material was centrifuged (20 min, 3300 rpm), and the supernatant containing isolated proteins was collected and transferred to a sterile Eppendorf tube. The pellet after centrifugation was seeded in the Columbia medium with sheep blood added (Difco) and incubated at 37°C for 18 hours to confirm that only proteins associated with the bacterial cell surface were isolated. Protein concentration was determined using the Pierce^{™} BCA Protein Assay (Thermo Fisher Scientific). Subsequently, the samples were aliquoted and stored at -20°C for further tests.

To separate the protein mixture, 10 µL of each sample was mixed with Laemmli buffer, reduced at 100°C for 5 min in a heating block, centrifuged at 13,000 g for 10 min and applied on the gradient polyacrylamide gel with a concentration of 4-20% [11]. Electrophoretic separation (SDS-PAGE) was performed initially at 80 V for 20 minutes and continued at 120 V for 40 minutes. Afterward, SDS-PAGE gel was washed three times in deionized water (diH₂O) and stained with Coomassie Brilliant Blue (BIO-RAD) or it was directly transferred into a polyvinylidene difluoride (PVDF) membrane (Millipore) at 135 V for 90 minutes.

Subsequently, the membranes were blocked in 1% bovine serum albumin (BSA) solution (Thermo Fisher) in PBS with Tween-20 (PBS-T, Thermo Fisher) added with shaking at room temperature for 1 hour.

Subsequently, the membranes were washed in PBS-T 3 times for 5 minutes, and incubated with the test serum solution at 37°C for 2 hours in a 1:1,000 dilution and washed again in PBS-T (3 x 5 min).

Incubation with horseradish peroxidase-conjugated sheep anti-bovine secondary antibodies (Novusbio) in a 1:10,000 dilution was performed at room temperature for 1 hour, and the membranes were washed in PBST 3 times for 5 min. Protein bands were visualized by inducing chemiluminescence using the Pierce^{™} ECL Western Blotting Substrate (Thermo Fisher Scientific) and then measured using the C-DiGit^{®} Blot Scanner (LI-COR).

Subsequently, 13 protein bands (3 for *E. coli,* 4 for *S. uberis,* 6 for *S. agalactiae*), identified among all the strains of the species but with absent (or very weak) immunoreactivity toward other species, were cut out from the gel and analyzed further to identify proteins.

### Protein identification

To identify the proteins cut out from the electrophoresis gel, the bands were destained with methanol, and then reduced and alkylated with dithiothreitol and iodoacetamide solutions. Subsequently, gel fragments were digested in trypsin solution in 50 mM carbonate buffer (Promega, Trypsin Gold, Mass Spectrometry Grade, Technical Bulletin) at 37°C overnight. The resulting peptides were extracted from the gel using a solution consisting of water, acetonitrile and trifluoroacetic acid with elution performed three times (v:v 45:50:5). The peptide mixture was purified and concentrated using C18 Zip-tips according to the manufacturer's instructions (Merck Chemicals, Billerica, MA, USA, PR 02358, Technical Note).

To identify proteins, the purified mixtures of test peptides with the solution of reference peptides were applied on the Anchor Chip MALDI plate (Bruker), on which 1 µL of α-cyano-4-hydroxycinnamic acid matrix (HCCA, Bruker) was subsequently applied.

Mass spectra of the analyzed samples were recorded in a range of 700-4,000 m/z using an Ultraflextreme MALDI TOF/TOF spectrometer (Bruker) with flexControl 3.3 software (Bruker). The resulting spectra were subjected to smoothing and cut-off lines were corrected. The list of peaks generated in flexAnalysis 3.0 (Bruker) for a signal-to-noise ratio greater than 3 was transferred to BioTools 3.2 (Bruker) and compared with Mascot 2.2 software (Matrix Science) using the Swiss-Prot database (www.uniprot.org) restricted to the "bacterial" taxonomy with a maximum error of 0.3 Da and cysteine carbamidomethylation as a mandatory modification and trypsin as the digestive enzyme.

The spectra were compared in terms of similarity to the peptide fingerprint specific for respective proteins. In this case, the number of overlapping peptide signals generated after digestion was used to compare the data with those found in the annotated protein database. The results obtained in Mascot with values greater than 61 were considered statistically significant (p ≤ 0.05). Other spectra of fragment ions for selected peptides were recorded in the LIFT mode and pooled to enable MALDI TOF/TOF identification. The results were interpreted as follows: a protein result was -10*Log(P), wherein P was the probability that the observed match was a random event. The higher score, the better identification.

### Bioinformatic analysis

To examine the structure, function and immunogenicity of the identified proteins, a number of bioinformatic analyses were performed including prediction of cell localization, antigenicity, detection of signal peptides and classic or non-classic proteins. In addition, selected proteins were analyzed to identify linear B cell epitopes, search for PDB models and identify conformational B cell epitopes.

Subcellular localization of the proteins was predicted using PsortB 3.0 and the Bologna Unified Subcellular Component Annotator (BUSCA) server, with differentiation between Gram-positive and Gram-negative bacteria. Subcellular localizations were annotated as GO, with extracellular annotated as GO:0005615 and cytoplasmic as GO:0005737.

Protein functions were predicted using PRED-LIPO (prediction of signal proteins), SignalP (classic proteins), Secretome (non-classic proteins for Gram-positive bacteria) and PncsHub (non-classic proteins for Gram-negative bacteria) servers. Classic or non-classic proteins were predicted with a default setting of 0.45 and 0.5, respectively.

*In silico* prediction using VaxiJen software was performed to identify the most antigenic proteins. Antigenic prediction was corrected to a default value of 0.4, and proteins having values ≥ 0.4 were considered antigenic.

B cell epitopes for antigenic proteins were identified using Antibody Epitope Prediction in the IEDB database. Conformational B cell epitopes were identified as follows: the PDB matrix was identified using SWISS-MODEL Workspace including PDB identifiers for each protein sequence. Subsequently, the best matrix (with higher identity) was selected, based on which a respective model was built. Selected PDB identifiers were then used to predict linear and discontinuous conformational B cell epitopes using ellipro software.

### Results

### Bacterial species identification

The species or genus of the etiological agent was confirmed for all isolates tested in the pool of milk specimens obtained from cows with confirmed mastitis. Bacteria of the genus *Streptococcus* were most frequently isolated (n=15; 68.2%), followed by bacteria of the species *Escherichia coli* (n=4; 18.2%), *Enterococcus cecorum* (n=1; 4.5%), *Aerococcus viridans* (n=1; 4.5%) and *Enterobacter cloacae* (n=1; 4.5%).

The species *Streptococcus uberis,* found in 11 test samples (73%), prevailed in the genus *Streptococcus, Streptococcus dysgalactiae* was detected in two samples (13%), and *Streptococcus agalactiae* was found in only one milk specimen tested (7%). One streptococcal strain (7%) was not identified as to the species.

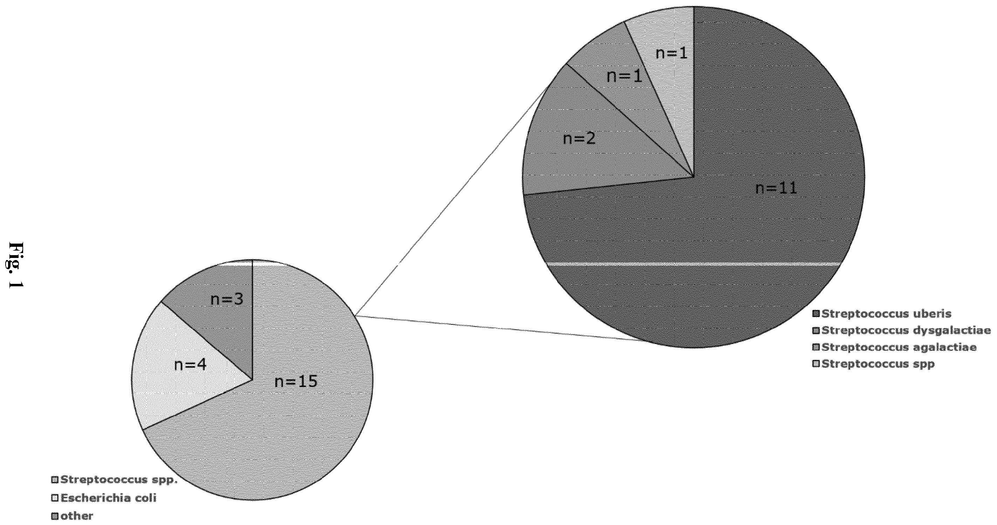

Fig. 1 shows the percentage of bacterial strains isolated from the milk of cows with confirmed mastitis, divided into species and/or genera.

### Protein detection

Protein detection was performed for three representative strains of *Escherichia coli,* three strains of *Streptococcus uberis* and one strain of *Streptococcus agalactiae.*

Culture of the pellet after protein isolation showed bacterial growth for each of the strains tested (qualitative assessment) and, therefore, it was assumed that the protein isolation procedure based on SDS did not damage bacterial cell walls; as a result, only proteins associated with the cell wall of the bacteria tested were collected.

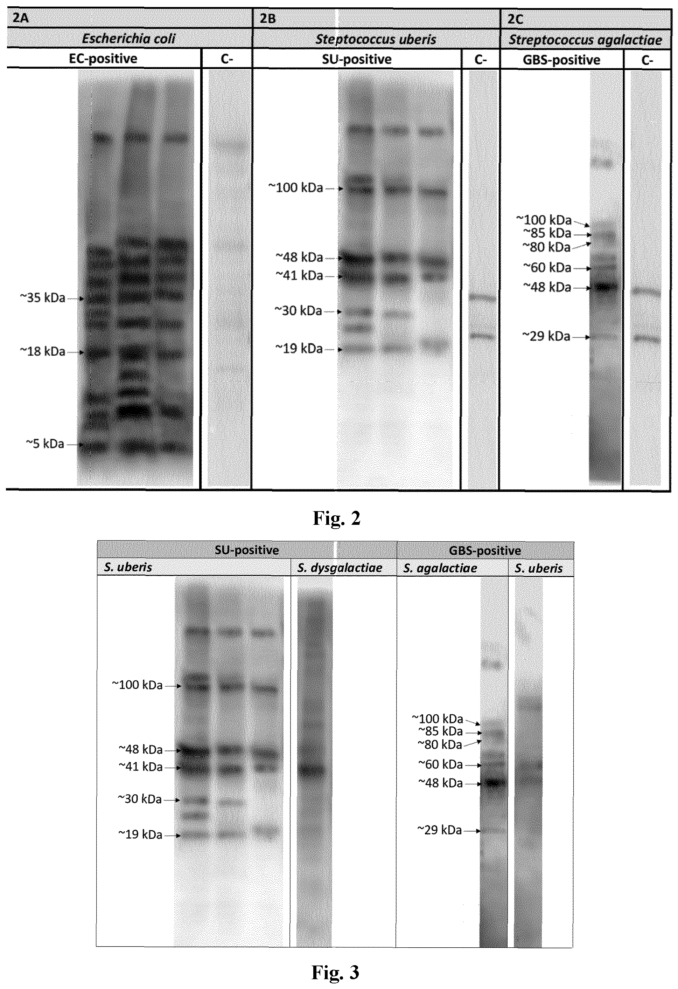

Fig. 2 shows examplary results of the immunodetection of proteins representative for *Escherichia coli, Streptococcus uberis* and *Streptococcus agalactiae* tested in the presence of sera from infected animals (EC-positive: sera from cows with mastitis caused by *Escherichia coli*; SU-positive: sera from cows with mastitis caused by *Streptococcus uberis,* GBS-positive: sera from cows with mastitis caused by *Streptococcus agalactiae* (group B streptococcus, GBS) and from healthy animals (C-).

From a pool of approx. 16 proteins immunoreactive in the presence of pooled EC-positive sera (sera from cows with mastitis due to *E. coli*: no. 1752, no. 0653/1) representative for *Escherichia coli,* three, of the following masses: approx. 5 kDa (EC1), approx. 18 kDa (EC2), and approx. 35 kDa (EC3), were further analyzed (Fig. 2A).

In addition, from a pool of approx. 14 *Streptococcus uberis* proteins specifically reacting with SU-positive sera (sera from cows with *S. uberis* mastitis: no. 0653, no. 5754, no. 7939) five bands with masses of approx. 19 kDa (SU4), approx. 30 kDa (SU3), approx. 41 kDa (SU5), approx. 48 kDa (SU1), approx. 100 kDa (SU2) (Fig. 2B) were cut out and subjected to sequencing.

The lowest protein immureactivity was reported for the species *Streptococcus agalactiae* (group B Streptococcus, GBS). This may have been caused by the lower number of sera (n=1, no. 1917) compared to the other species evaluated. Ultimately, 6 proteins with the following masses: approx. 29 kDa (GBS1), approx. 48 kDa (GBS2), approx. 60 kDa (GBS3), approx. 80 kDa (GBS8), approx. 85 kDa (GBS4) and approx. 100 kDa (GBS7) were selected for further analysis (Fig. 2C).

It is noted that the proteins listed above showed no immunoreactivity (or their reactivity was very low) with sera from cows without mastitis (negative sera), which confirmed their high specificity. Instances of any reactivity may have been due to previous contact with the test pathogens. In addition, cross-reactivity between the streptococcal sera tested was also not seen for most protein bands (*S. uberis, S. agalactiae, S. dysgalactiae*).

Fig. 3 shows examplary results for the cross-reactivity between *Streptococcus agalactiae, Streptococcus uberis* and *Streptococcus dysgalactiae* tested in the presence of sera from cows with mastitis caused by *Streptococcus agalactiae* (GBS-positive) and pooled sera from cows with *Streptococcus uberis* mastitis (SU-positive).

### Protein identification

The resulting mass spectra of the proteins were compared with data available in Swiss-Prot. As a result, 13 proteins were identified for a significance threshold of p≤0.05: six were identified for *S*. *agalactiae*: aspartate carbamoyltransferase (GBS 1), elongation factor Tu (GBS 2), 60 kDa chaperonin (GBS 3), elongation factor G (GBS 4), galactose-6-phosphate isomerase subunit LacA (GBS 7) and adenosine deaminase (GBS 8), four for *S. uberis*: elongation factor Tu (SU 1), tRNA uridine 5-carboxymethylaminomethyl modification enzyme MnmG (SU 3), GTPase Obg (SU 4), glyceraldehyde-3-phosphate dehydrogenase (SU 5), and 3 for *E. coli*: molybdenum cofactor biosynthesis protein B (EC 1), aldehyde reductase YahK (EC 2) and outer membrane protein A (EC 3). Differences in molecular masses between the mass of the identified proteins and that determined by SDS-PAGE based on a comparison with the protein marker mass may result from multiple protein forms present, their modification or their partial breakdown.

### Bioinformatic analysis

Thirteen of the proteins listed above were subjected to bioinformatic analysis to determine their immunogenicity and functionality. In the first step of the analysis, the proteins were classified based on their cell localization. As a result, 12 proteins were classified as cytoplasmic (GO: 000573), and one (EC3) was localized in the outer membrane (GO: 0019867) of subcellular elements.

Subsequently, the proteins were divided into classic and non-classic. As a result, 9 proteins: EC3, GBS 1, GBS 2, GBS 3, GBS 4, GBS 7, SU 1, SU 3, SU 5 were identified as non-classic proteins (secretomic proteins). Furthermore, the proteins were also classified in terms of their antigenic potential and 12 of the 13 proteins were identified as antigens. Detection of signal peptides showed that only one protein (EC3) had a signal peptide (Sec signal).

The structures of each of the 13 proteins were analyzed to identify B cell epitopes. As a result, these were confirmed for all proteins, and their number ranged from 1 to 22. This, in turn, facilitated further analysis that included identification of conformational epitopes for all the proteins tested. The most similar structure of a PDB (reference) protein was identified first, based on which a model of the investigated protein was built. Matrix identity for the whole pool of analyzed proteins was in a range of 35-100%. The models thus built were used to identify linear and discontinuous conformational epitopes. As result, 2-10 linear epitopes and 3-8 discontinuous epitopes were found. The results are shown in Table 1.

### Identification of antigens specific for pathogenic Staphylococcus aureus strains responsible for mastitis in cows

The study included 3 representative *Staphylococcus aureus* strains isolated from the milk of cows with confirmed mastitis in the proprietary collection of the West Pomeranian University of Technology in Szczecin.

In addition, 12 serum samples were a pool of negative controls, collected by veterinarians from the Omniwet Veterinary Clinic in Orzesze within routine tests to determine standard clinical chemistry parameters.

All isolates were stored in the Microbank^{™} Preservation System (BioMaxima S.A., Poland) at -80°C for further testing. The serum samples were stored in the same temperature conditions.

### Methods

### Bioinformatic analysis

To identify the highly specific surface proteins representative for *Staphylococcus aureus* potentially found in cow's milk, which could serve as specific biomarkers in the innovative immunochromatographic test for diagnosing mastitis, the following analyses were performed.

In the first stage, amino acid sequences were retrieved from Esembl Bacteria and UniProt databases. Due to the very large volume of data, the sequences were merged into single files representative for respective species. Subsequently, the sequences were divided into two groups: "positive" (Gram-positive) and "negative" (Gram-negative). At the next stage, the amino acid sequences were clustered using CD-HIT software. Analyses were performed for the identity thresholds of 70%, 80% and 90%. In addition, potential surface proteins were predicted using PSORTb software which determined protein localization in the bacterial cell. The test sequences were divided into: "Cellwall" (associated with the cell wall), "CytoplasmicMembrane" (found in cytoplasm), "Extracellular" (extracellular proteins) and "Unknown". Only the sequences identified as "CellWall" were selected for further analysis. At the next stage, the data obtained through analyses using PSORTb and CD-HIT were merged. The amino acid sequences representative for surface proteins from *Staphylococcus aureus,* classified into a common cluster with the sequences representative for other Gram-positive species, were excluded from further analysis. The other proteins were analyzed in the NCBI BLAST database to compare them with reference sequences representative for a total of 18 bacterial, viral and fungal species that could potentially be found in cow's milk. Only the sequences not identifiable in any way with the reference sequences were selected for further analysis. Within the last stage of bioinformatic analysis, the amino acid sequences not excluded in previous stages were analyzed in the BLASTp database in terms of the detection of non-redundant (NR) protein sequences. Finally, only the sequences unique for the bacterial species tested (with a maximum identity threshold 75%) were selected.

### Bacterial culture

To revive the *S. aureus* strains from the bank, 100 µL of each milk sample was cultured in the Columbia medium with sheep blood added (Difco) in aerobic conditions at 37°C for 24 hours. Afterward, a single *S. aureus* colony was collected for further study and cultured in 9 mL of liquid tryptic soy medium in aerobic conditions at 37°C for 24 hours.

### DNA isolation

Bacterial DNA was isolated using a commercial Genomic Mini Kit (A&A Biotechnology) according to the manufacturer's procedure modified by adding 0.4 U/µL lysostaphin (A&A Biotechnology). The concentration and purity of the isolated DNA was evaluated using the NanoDrop device (Thermo Scientific). The DNA was stored at -20°C for further tests.

### fnbA gene detection

The PCR to detect the *fnbA* gene encoding fibronectin binding protein A (FnBPA) was performed based on a procedure developed by Tristan et al. [12] with minor modifications. The ***fnbA*** gene was amplified using primers with the following sequences: *fnb*A-F (CATAAATTGGGAGCAGCATCA) and *fnb*A-R (ATCAGCAGCTGAATTCCCATT) (Genomed) at 0.1 µM (1.25 µL of each primer per sample), 12.5 µL of the commercial PCR Mix Plus Kit (A&A Biotechnology) and 7.5 µL RNase-free water (A&A Biotechnology) and 2.5 µL bacterial DNA. The PCR was performed in the T100 Thermal Cycler (BIO-RAD) according to the following program: initial denaturation (5 min, 94°C), 30 amplification cycles including steps of denaturation (1 min, 94°C), primer annealing (1 min, 55°C) and extension of DNA strand (1 min, 72°C). The reaction was completed with a step of 10-minute incubation at 72°C. The amplification product with a size of 128 bp was visualized after 60-minute electrophoresis in 1% agarose gel (Prona).

### Protein isolation

Two methods were compared to optimize the isolation procedure for surface proteins of *Staphylococcus aureus.*

The first method is described in detail in section **"Protein isolation and detection".**

Furthermore, according to the second procedure reported by Banner et al. [13], bacteria were cultured in the liquid TSB medium in aerobic conditions at 37°C for 18 hours, and the concentration of bacterial suspension was brought to 3.3 McFarland in PBS (Gibco). The bacteria were washed twice and centrifuged (4000 x g, 3 min). Subsequently, the supernatant was removed and the pellet was suspended in 150 µL of PBS solution with 12 µL lysostaphin (A&A Biotechnology) and 1 mM PMSF protease inhibitor (phenylmethylsulfonyl fluoride; BIO-RAD) added and incubated at 37°C for 30 min with shaking. Subsequently, the material was centrifuged (4000 x g, 20 min, 4°C), and the supernatant was collected.

Protein concentration was determined using the Pierce^{™} BCA Protein Assay (Thermo Fisher Scientific). Subsequently, the samples were aliquoted and stored at -20°C for further studies.

### SDS-PAGE and immunoblotting

10 µL of the surface protein mixture was separated according to Laemmli's procedure [11]. After reduction at 100°C for 5 minutes, the samples were centrifuged for 15 minutes. SDS-PAGE was completed at a constant current of 25 mA. Subsequently, the gels were rinsed three times in deionized water and stained with Coomassie Brilliant Blue (BIO-RAD) according to the manufacturer's procedure or were directly transferred onto the PVDF membrane, at a voltage of 135 V for 90 minutes.

The membranes were fixed in 1% BSA solution (Sigma-Aldrich) in PBS-T (Thermo Fisher) at 4°C overnight. The membranes were washed three times in PBS-T for 5 minutes and incubated with anti-FnBPA monoclonal antibodies (Biolim) diluted in PBS-T at 22°C for 1 hour in a concentration of 1:100, and washed again (3 x 5 min). Incubation with peroxidase-conjugated goat anti-mouse secondary antibodies (Thermo Fisher) and/or horseradish peroxidase-conjugated sheep anti-bovine secondary IgG antibodies (Novusbio) diluted in PBS-T in a concentration of 1:5,000 was performed at 22°C for 1 hour. Subsequently, the membranes were washed 3 times in PBS-T for 5 minutes, and bands were visualized using 1-Step TMB Solution (Thermo Fisher) for 20 minutes. The reaction was stopped by immersing the membrane in diH2O.

### Results

### Bioinformatic analysis

Bioinformatic analysis was performed for a total of 515 species and 93,800,000 amino acid sequences retrieved using Ensembl Bacteria and UniProt programs, of which 292 species and 46,180,426 amino acid sequences were classified in the "Gram-positive" group. Using the CD-HIT program, 545,500 clusters were generated corresponding to Gram-positive bacteria at an identity level of 70%. In addition, PSORTb was used to detect 101 proteins representative for *Staphylococcus aureus* localized on the cell surface, of which 74 can potentially be found in milk, as predicted based on analysis performed in the NCBI BLAST database. Ultimately, 36 surface proteins representative for *Staphylococcus aureus* were identified, and fibrinogen binding protein (FnBPA) was one of these.

### fnbA gene detection

As a result of a PCR, the *fnb*A gene was confirmed in all the clinical strains of *Staphylococcus aureus* tested.

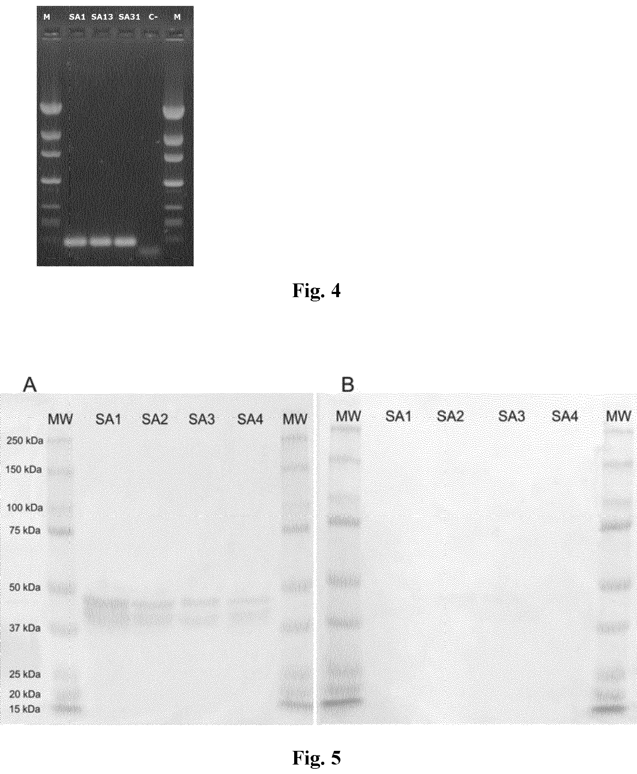

Fig. 4 shows *fnb*A gene detection (128 bp). Legend: M: marker; SA1, SA13, SA31: *S. aureus* strains tested, C-: negative control.

### SDS-PAGE and immunoblotting

The quantity, arrangement and quality of bands obtained using the two isolation methods showed that the second method (using lysostaphin) was slightly better and, therefore, it was selected for further experiments.

An FnBPA protein fragment of an appropriate size (approx. 40 kDa) was detected and immunoreactivity in the presence of anti-FnBPA monoclonal antibodies was tested to confirm specificity in the presence of sera from healthy cows; as a result, the protein was confirmed in all the *Staphylococcus aureus* strains tested.

Fig. 5 shows FnBPA protein detection for *S. aureus* in the presence of anti-FnBPA monoclonal antibodies (A) with confirmation of its specificity in the presence of sera from healthy cows (negative control) (B). Legend: MW: molecular weight marker (BIO-RAD); SA1-SA4: protein lysates of cow *S. aureus* isolates.

Table 1 shows the amino acid sequences identified according to the invention of epitopes from the protein antigens specific for pathogenic *Staphylococcus aureus* strains causing mastitis in cows identified according to the invention.

### Example 2. A diagnostic test for detecting mastitis caused by E. coli, S. uberis, S. agalactiae or S. aureus

### Diagnostic kit

In an example embodiment of the diagnostic composition of the invention, the diagnostic test for detecting mastitis caused by *E. coli, S. uberis, S. agalactiae* or *S. aureus* based on the composition consists of the following components:

### OUTER:

A) plastic case
B) window for applying a milk sample

### INNER:

A) **pad for sample application:** cellulose fiber strips, thickness: 0.83 mm, weight: 291 g/m2, flow rate: 196 mL/min and retention: 18 µm (e.g. C083 Cellulose Fiber Sample Pad Strips; cat. no.: CFSP173000, Millipore);
B) **pad with conjugate with antibodies:** fiberglass diagnostic pad, strips with a thickness of 0.41 µm and weight of 0.75 g/m2 (e.g. Glass Fiber Diagnostic Pad 10 mm x 300 mm strips 100 pk, cat. no.: GFDX103000, Millipore);
C) **test membrane** with the following: control band and 4 test bands with immobilized monoclonal antibodies that specifically recognize the protein antigens found in the cells of bacterial species detected, laminated cards with dimensions of 6 cm x 30.1 cm with a HI-Flow^{™} Plus lateral flow membrane and backing with 2 µm thickness glued to the card surface, e.g. HI-Flow^{™} Plus HF075 Membrane Cards (cat. no.: HF075MC100, Millipore) with a high flow rate; HI-Flow^{™} Plus HF 120 Membrane Cards (cat. no.: HF120MC100, Millipore) with an intermediate flow rate; HI-Flow^{™} Plus HF 180 Membrane Cards (cat. no.: HF180MC100, Millipore) with a low flow rate;
D) **pad for absorbing unbound reagents:** cellulose fiber strips, thickness: 0.83 mm, weight: 291 g/m2, flow rate: 196 mL/min and retention: 18 µm, e.g. C083 Cellulose Fiber Sample Pad Strips (cat. no.: CFSP173000, Millipore)

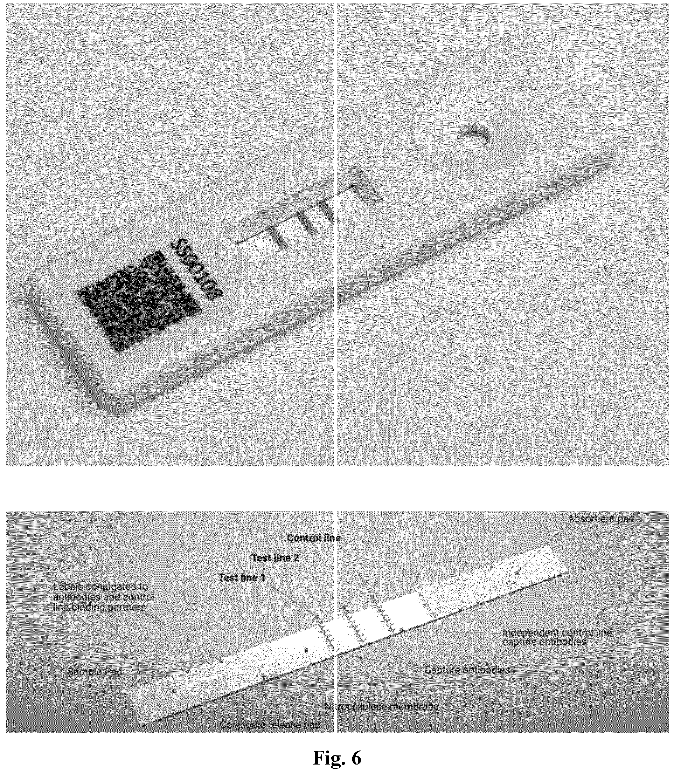

Fig. 6 shows a diagram of the layout and operation of the immunochromatographic test.

### Testing procedure and interpretation:

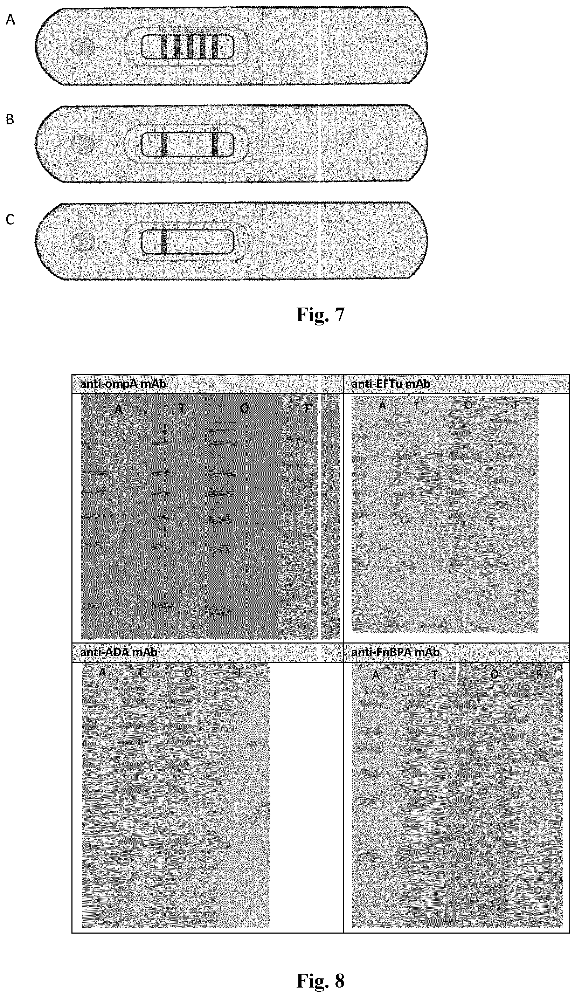

**1.** A milk sample is applied at the start of an appropriately marked band (sample pad) using a sterile pipette
**2.** As a result of capillary forces, the test sample moves toward the pad containing a conjugate of antibodies with colloidal gold (conjugate pad) on which they bind (antigen-conjugate)
**3.** Antigen-conjugate complexes of colloidal gold move through the test band (migration membrane) for 10-20 minutes, on which 4 types of monoclonal antibodies are immobilized (anti-ompA, anti-EF-Tu, anti-ADA, anti-FnBPA) that specifically recognize one of four antigens (ompA, EF-Tu, ADA and/or FnBPA) (test line). Appropriate monoclonal antibodies can be obtained using any known technique, for example through the immunization of mice with proteins produced in *E. coli.* One band corresponds to one tested antigen. In an example embodiment, the antibodies were obtained using a procedure based on the following stages: immunization, fusion, production of antibodies and determination of antibody titer, according to the procedure reported by Galfrè and Milstein [14]. Depending on the protein based on which species are differentiated, the band is found at a different height (Fig. 7).

### Interpretation:

After the complex of the test antigen with antibodies forms, a color band develops on the membrane, indicating a positive result. When at least two bands (test and control) develop, a positive result is obtained (mastitis caused by *S. aureus* or *S. agalactiae* or *S. uberis* or *E. coli),* and one band (control band) indicates a negative result of a test performed correctly. No band or a test band only indicates that the test has been performed incorrectly and the whole procedure will need to be performed again. Test interpretation is completed quickly and it is unambiguous and can be performed both by a veterinarian and by a person responsible for monitoring animal health in a herd.

Fig. 7 shows a diagram of the immunochromatographic test of the multiplex type for the simultaneous identification of *Staphylococcus aureus* (SA), *Escherichia coli* (EC), *Streptococcus agalactiae* (GBS) and *Streptococcus uberis* (SU). Legend: A: positive test for all the species tested; B: positive test for *Streptococcus uberis*; C: band to confirm that the test has been performed correctly (source: internal materials).

### Example 4. Determination of the titer of monoclonal antibodies as a component of the developed immunochromatographic test and antigens detected in the test specimen and a test to rule out cross-reactivity

The objective of the first stage was to rule out cross-reactivity between the components of sterile milk in the presence of monoclonal antibodies (anti-ompA, anti-EFTu, anti-ADA, anti-FnBPA) using Western blot and ELISA assay techniques. Based on the techniques, unspecific reactions between the monoclonal antibodies and the components of milk obtained from cows without confirmed mastitis were ruled out.

Subsequently, using Western blot, reactivity between recombinant proteins (ompA, EFTu, ADA i FnBPA) and monoclonal antibodies (anti-ompA, anti-EFTu, anti-ADA, anti-FnBPA) was tested. Protein electrophoresis was performed on polyacrylamide gel using the SDS-PAGE technique and subsequently using immunoblotting in the presence of the monoclonal antibodies in a concentration of 1:100 diluted in PBST (Fig. 8).

Fig. 8 shows an example result of a cross-reactivity test between the antigens (recombinant proteins, (A: ADA, T: EF Tu, O: ompA, F: FnBPA) and monoclonal antibodies (anti-ompA, anti-EFTu, anti-ADA and anti-FnBPA).

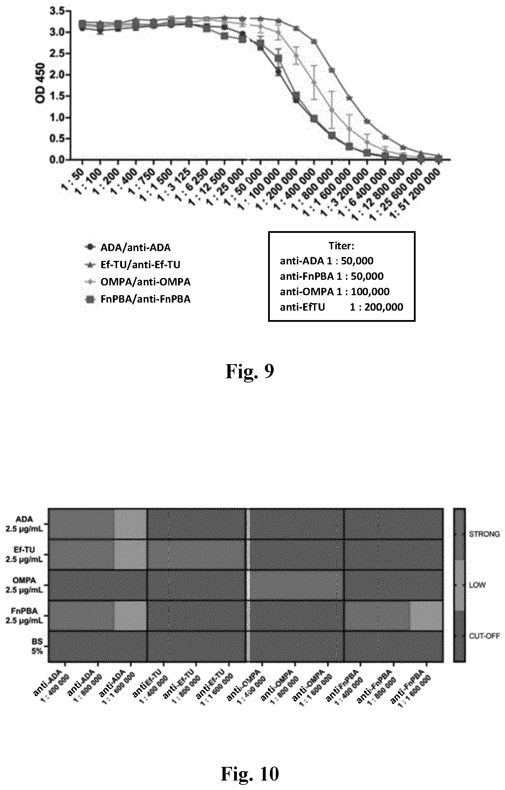

In the subsequent stage, antibody titers were determined using the ELISA assay. Therefore, wells of a 96-well MaxiSorp plate were coated with proteins in a concentration of 2.5 µg/mL at 4°C for 16 hours. Subsequently, unbound antigens were rinsed off 3 times in TBST. The wells were blocked with a blocking buffer and dilutions of the anti-ADA, anti-EFTu, anti-ompA and anti-FnBPA antibodies were prepared using a diluent solution (2% skimmed milk in PBS) in a geometric range between 1:25 0000 and 1:51 200 000, and 100 µL of an appropriate solution of the antibodies was applied into each well and incubated at 4°C for 2 hours. Unbound antibodies were rinsed off 3 times in the TBST buffer. Subsequently, 100 µL of horseradish peroxidase-conjugated anti-mouse antibodies diluted in the diluent solution in a 1:5,000 ratio were applied on the wells and incubated at 4°C for 1 hours, and the wells were washed 3 times in the TBST buffer. The reaction product was visualized by incubation with the TMB solution at room temperature for 20 minutes. The color reaction was stopped by adding 50 µL of the Stop Solution. Absorbance was measured at wavelengths of 450 and 570 nm, and results for three measurements for each sample were processed in GraphPad Prism 9 by Dotmatics. The experiments to determine antibody titers were performed in two stages. In the first stage, in which concentrations from 1:50 were tested, the lowest titer of antibodies that reacted with the proteins could not be determined. Failure of determination of the threshold value could have resulted from a high concentration of the antibodies prepared by the manufacturer, that is, 3-4 mg/mL. In the subsequent stage, the ranges of concentrations tested were expanded to between 1:250,000 and 1:51,200,000. As a result of the experiments, the titer was determined, defined as the first decrease in the absorbance after the plateau, and it was thus shown that the optimal titers of monoclonal antibodies for anti-ompA were 1:100,000, 1:200,000 for anti-EFTu, 1:50,000 for anti-ADA and 1:50,000 for anti-FnBPA (Fig. 9).

Fig. 9 shows a titration result for the anti-ompA, anti-EFTu, anti-ADA and anti-FnBPA antibodies in the presence of antigens (ompA, EF-Tu, ADA, FnBPA) in a quantity of 0.25 µg/well.

Fig. 10 shows results of testing potential cross-reactivity between antigens (ADA, EF-Tu, ompA, FnBPA) and monoclonal antibodies (anti-ADA, anti-EFTu, anti-ompA, anti-FnBPA) using the ELISA assay with different concentrations of the monoclonal antibodies. In addition, a cut-off value of 0.016 was determined based on the test.

In the subsequent stage, binding capacity of the antibodies to whole bacterial cells was tested. To select an appropriate antigen concentration for the Western blotting technique, a cELISA assay was used for testing the following species: *E.coli, K. pneumoniae, Ps. aeruginosa, S. aureus, E. faecalis, S. agalactiae, S. uberis,* suspended in PBS with 4% formaldehyde in concentrations corresponding to OD₆₀₀ values of 2.0, 1.0, 0.5 and 0.1. It was found that antibody sensitivity declined with decreasing OD values, but the results were above the detection threshold for all the concentrations tested. Bacterial lysates with concentrations corresponding to OD₆₀₀=0.1 and OD₆₀₀=1.0 were selected for further studies using the SDS-PAGE technique. The proteins were isolated with 0.1% SDS at 42°C. Considering the low quantity of bands visualized using Coomassie stain, it was decided to modify the isolation procedure.

Therefore, two procedures were tested. According to the first procedure, bacterial suspension with optical density OD₆₀₀=1.0 in a volume of 1 mL was centrifuged for 10 min at 4500 RPM, and subsequently suspended in 200 µL lysis buffer consisting of 0.2% SDS, 0.1% Triton X-100, 50 µg/mL lysozyme and 1 µL benzonase. The lysates were incubated at 25°C for 30 minutes and subsequently at 42°C for 1 hour with constant vigorous shaking (700 RPM). In the second procedure, 2 milliliters of bacterial suspension with optical density OD₆₀₀=2.0 was centrifuged for 10 min at 4500 RPM, and subsequently suspended in 150 µL lysis buffer A, consisting of 100 µg/mL lysozyme, 1 µL benzonase and 50 U mutanolysin. After preincubation for 30 minutes at 25°C with constant vigorous shaking (700 RPM) 150 µL lysis buffer B, consisting of 0.4% SDS, was added to each sample and incubated at 42°C for 30 min with constant vigorous shaking (700 RPM). Based on the analysis of the quantity of protein bands obtained in the isolation according to the hereinabove procedures, the second procedure using mutanolysin was selected.

After the end of isolation, the quantity of proteins isolated was determined using the Bradford assay. Therefore, 10 µL of bacterial lysate was incubated with 250 µL of the Bradford reagent (Thermo Scientific Chemicals), and the samples were incubated in darkness for 10 minutes. Absorbance values for respective samples were measured at a wavelength of 595 nm. Absorbance results were referred to the standard curve obtained based on successive BSA dilutions in a range of 0.00-1.50 mg/mL. Quantities of the resulting proteins were in a range of between 9.5 and 36.9 µg for samples in a volume of 20 µL.

After the isolation procedure for bacterial proteins was standardized, the lysates were tested to evaluate the binding capacity of the monoclonal antibodies to bacterial cell lysates using Western blot. Therefore, the resulting bacterial lysates were separated using the SDS-PAGE technique. Bacterial lysates diluted in PBS with a final protein concentration of 20 µg/mL were used for the separation. 4 µL of a 5x concentrated sample buffer (Thermo Fisher) was added to the samples and incubated at 95°C/5 min and separated by SDS-PAGE in four replicates.

After separation, the proteins were transferred onto nitrocellulose membranes for Western blot. After transferring, the membranes were blocked with the Blocking Solution (Thermo Fisher) for 1 hour with shaking, and the membranes were subsequently coated (separately) with the antibodies: anti-ADA, anti-EfTU, anti-OMPA, anti-FnPBA in the following titers: 1:50,000; 1:50,000; 1:100,000; 1:200,000 and incubated at 4°C for 16 hours with constant shaking. After incubation, the membranes were washed three times for 10 min using PBS buffer containing 0.05% Tween-20 (PBST). Subsequently, the membranes were coated with horseradish peroxidase (HRP)-conjugated goat anti-mouse antibodies (Invitrogen) with a titer of 1:5,000 and incubated at 4°C for 2 hours with constant shaking. The membranes were washed three times for 10 min using PBST buffer. Subsequently, a substrate (3,3',5,5'-tetramethylbenzidine, TMB, Thermo Fisher) was added onto the membranes until completely coated and incubated at 4°C in darkness for 20 min. Thus prepared membranes were visualized.

It has been shown that the anti-EfTU antibodies for detecting *S. uberis* bound all the reference *S. uberis* strains selected to standardize the procedure.

### Example 5. Preparation of monoclonal antibodies conjugated with gold nanoparticles

### Production of gold nanoparticles

Gold nanoparticles with a diameter of 30 nm were synthesized in a chemical reaction involving reduction of tetrachloroauric(III) acid (Thermofisher) using sodium citrate (Chempur). Before starting the reaction, laboratory glassware was cleaned chemically using aqua regia prepared by mixing 3 parts of hydrochloric acid with 1 part of nitric(V) acid.

To synthesize the nanoparticles, 0.01% tetrachloroauric(III) acid solution was brought to a boil and subsequently 1 mL of 1% sodium citrate was added while maintaining the boiling temperature for 10 minutes with constant vigorous stirring. The color of the solution changed from yellow through gray to intense burgundy red color during the reaction. To prevent excessive evaporation of the solution, the reactions were completed using cooling equipment. After the end of the reaction, the solution was cooled to room temperature by switching off the heating. 100 mL of gold nanoparticle solution was prepared and subjected to UV-VIS and DLS (Dynamic Light Scattering) analysis in five replicates to determine their size and estimate the properties of the colloid system, that is, monodispersity. As a result, 100 mL of stable monodisperse gold nanoparticle solution with a size of 30 nm was obtained.

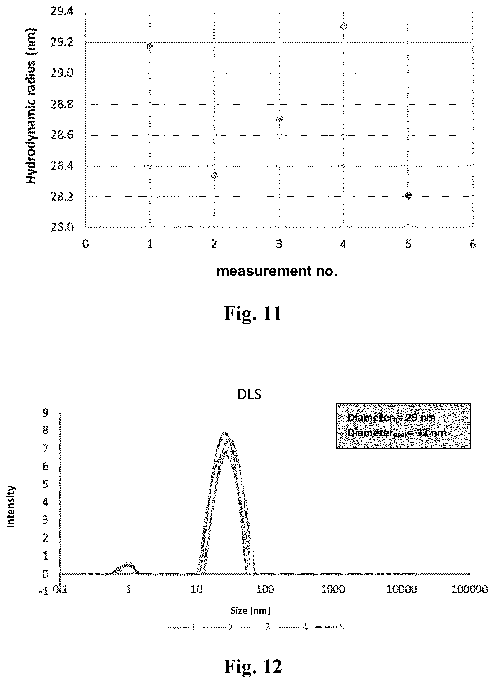

Fig. 11 shows the results of hydrodynamic measurements of the resulting gold nanoparticle solution in five replicates.

Fig. 12 shows the results of DLS (Dynamic Light Scattering) measurements of the gold nanoparticle solution prepared in five replicates by reducing tetrachloroauric(III) acid using sodium citrate.

### Conjugation of monoclonal antibodies with gold nanoparticles

A conjugation procedure between the resulting gold nanoparticles (AuNps) and anti-ompA, anti-EFTu, anti-ADA, anti-FnPBA monoclonal antibodies (mAbs) (Biolim) was performed.

In the first step, the pH of aqueous AuNps solution was set using 0.2 M K₂CO₃ (potassium carbonate, Merck) to pH=7.5. Subsequently, a microscale stability test of mAb conjugation with AuNps was performed. Therefore, the following series of mAb dilutions were prepared: 40, 20, 10, 5, 2,5, 1,25 µg/mL using ultrapure water buffered with K₂CO₃ of pH=7.5. Adequate mAb quantities were added to 100 µL AuNps to obtain the concentrations and the systems were incubated at room temp. for 1 hour to bind mAbs with AuNps. After incubation, 50 µL of 1 M NaCl each was added and incubated at room temp. for 30 min. UV-VIS measurements in a wavelength range of 250-900 nm were performed. Based on this, an optimum concentration of each mAb for conjugation with AuNps was determined, at which the AuNps solution remained stable (no aggregates or precipitation of AuNps from the solution). The optimum concentrations of anti-ompA, anti-EFTu, anti-ADA, anti-FnPBA mAbs used for conjugation with AuNps are 20, 10, 10, 20 µg/mL, respectively. In addition, the success of mAb conjugation with AuNps could be determined based on the measurements in this range. Due to the conjugation of mAbs with AuNps, the size of AuNps compared to the original (unconjugated) colloid changed. The charts provided below show the mAb+AuNps absorbance value shifted compared to the control, that is, pure AuNps and AuNps with 1 M NaCl added.

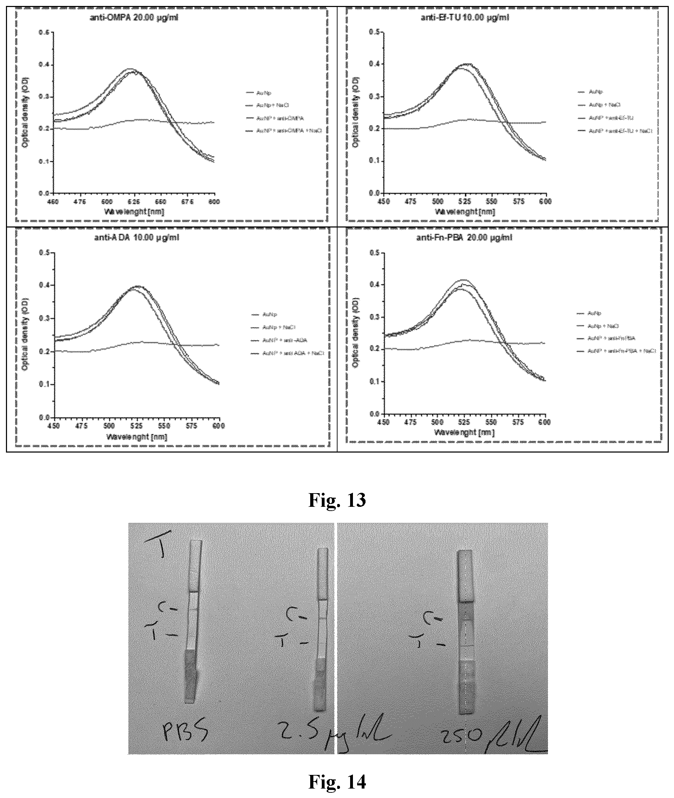

Fig. 13 shows charts presenting AuNps spectra after conjugation with mAbs.

In the second stage, mAb conjugation with AuNps on a larger scale was performed based on the above results. Therefore, dilutions of anti-ompA, anti-EFTu, anti-ADA, anti-FnPBA mAbs were prepared in concentrations of 20, 10, 10, 20 µg/mL, respectively, in 2 mL of borate buffer and pH=8.5. The resulting dilutions were added to 10 mL AuNps and incubated at 4°C for 1 hour. After incubation, 1.5 mL of 10% BSA solution prepared from 20 mM borate buffer was added and incubated at 4°C for 15 min. Subsequently, the systems were centrifuged at 2,500 x g at room temperature for 30 min, the supernatant was removed and it was washed twice with 20 mM borate buffer containing 1% BSA. The precipitate was finally suspended in 1 mL of 20 mM borate buffer and added to 1 mL suspension buffer containing 3% sucrose, 0.6 M NaCl, 0.2% Tween-20, 2% BSA, prepared from 20 mM borate buffer.

### Example 6. Performing the lateral flow assay

The following materials were used in the example embodiment shown below:
**Sample pad:** fiberglass material (Glass Fiber Diagnostic Pad, Merck) impregnated with pH 7.2 PBS with 0.2% Tween-20, 0.1 M NaCl added, dried at 50°C for 30 min.
**Conjugate pad:** fiberglass material (Glass Fiber Diagnostic Pad, Merck). 20 µL/cm of AuNP conjugated with mAbs (OD = 4) suspended in 20 mM borate buffer of pH 8.0, 3% sucrose, 0.6 M NaCl, 0.2% Tween-20, 2% BSA was applied each on the material.
**Nitrocellulose membrane:** nitrocellulose material (Hi Flow Pus HF180 membrane card, Merck) was applied on two lines:
   I) Test line: mAb against bacterial proteins, 1 mg/mL. Size of the line applied: 1 µL/cm,
   II) Control line: mAb against mouse antibodies, concentration: 0.5 mg/mL. Size of the line applied: 1 µL/cm, interval between the lines: approx. 0.5 cm. The membranes thus prepared were blocked with 1% BSA until the whole membrane surface was coated and subsequently dried at 37°C for 12 h.
**Absorbent pad:** cellulose material (Cellulose fiber sample pad, Merck).

The lateral flow assay was assembled as follows: The sample pad overlapped with its surface onto the conjugate pad which overlapped with the start of the nitrocellulose membrane. The absorbent pad was placed at the end of the nitrocellulose membrane to absorb any excess liquid. Test bands with a width of 30 mm were cut out from the membranes thus prepared.

In the first stage, freshly diluted freeze-dried proteins ompA, ADA, FnBPA and EF-Tu were diluted to a concentration of 1 mg/mL (stock), and two dilutions were subsequently prepared for each protein from the original stock: 2.5 µg/mL (concentration selected based on completed ELISA and WB tests) and 250 µg/mL (100 µL each for a test). PBS was the negative control. It was found in the experiments that the test worked because a band appeared on the control line (antibodies against mouse antibodies), while no evident test bands were seen (Fig. 14), which could indicate that the concentration of monoclonal antibodies applied on the test membrane was too low.

Fig. 14 shows an examplary result of the lateral flow test for detecting EF-Tu protein representative for *S. uberis.* Legend: T: EF-Tu protein, C: control band, T: test band, PBS: negative control with PBS, 2.5: test for protein in a concentration of 2.5 µg/mL, 250: test for protein in a concentration of 250 µg/mL.

In addition, whole bacterial cells representative for 4 test species *(S. uberis, S. aureus, E. coli, S. agalactiae*) were tested with 3 different OD values: 1.0, 0.1, 0.01). 0.8% NaCl was the negative control. However, positive results were not reported for test strips in this experiment, which indicated that mAb concentration had to be increased.

Subsequently, the experiments reported above were repeated: both with recombinant proteins (at a concentration of 250 µg per test) and with whole live bacterial cells (example isolates at OD 1.0, 0.1 and 0.01) and bacterial lysate (OD=1.0), at an increased concentration [control strip, 1 mg/mL, test strips, 1 mg/mL (with application 3 times)]. The results showed that the test worked correctly (the control appeared); in addition, the test band appeared for EF-Tu protein for *S. uberis.* An identical result was obtained for an example bacterial lysate from *S. uberis.*

In the subsequent stage, the test based on anti-EfTU antibodies was validated in the presence of 203 cow's milk samples from three regions of Poland, of which 100 were obtained from cows with a clinical type of mastitis (study group), and 103 were obtained from cows without clinical symptoms of mastitis (control group). Subsequently, the test results were subjected to statistical analysis to determine sensitivity, specificity and accuracy compared to the gold standard, that is, culture results in the chromogenic Chromagar mastitis medium. It was found in the analysis that the test prototype developed had specificity of 89.02%, accuracy of 79.31% and sensitivity of 38.46% for milk samples containing bacteria of the genus *S. uberis* with CFU > 1 x 10³.

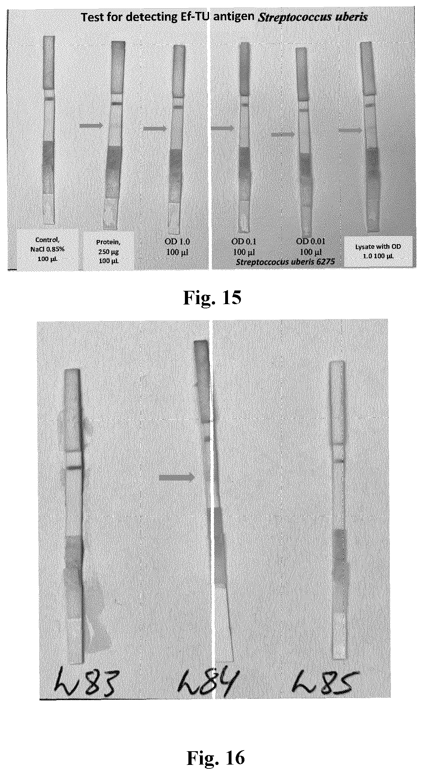

Fig. 15 shows the result of a test for detecting the *S. uberis* EF-Tu antigen with recombinant EF-Tu, proteins, bacterial lysates from the species *S. uberis* and whole bacterial cells as examples.

Fig. 16 shows an example positive result of the test *S. uberis* in a milk sample (W84) based on monoclonal anti-EfTU antibodies.

**Table 1. List of epitopes for selected antigens representative for E. coli (ompA), S. uberis (EF-Tu), S. agalactiae (ADA) and S. aureus (FnBPA).**

| **No.** | **Sequence** | **Length (number of amino acids)** |
|---|---|---|
| **ompA protein** | | |
| 5. | ³⁶WSQYHDTGFINNNGPTHENQLGAG⁵⁹ | 24 |
| 6. | ⁷⁶YDWLGRMPYKGSVENGAY⁹³ | 18 |
| 7. | ¹⁰⁵GYITD¹¹⁰ | 6 |
| 8. | ¹²⁶DTKSNVYGKNHDTGVSP¹⁴² | 17 |
| 9. | ¹⁵⁵EIATRLEYQWTNNIGDAHTIGTRPDNGMLSLGVSYRFGQG EAAPVVAPAPAPAPEVQTKHF²¹⁵ | 61 |
| 10. | ²²²LFNFNKATLKPE²³³ | 12 |
| 11. | ²⁶⁴IGSDAYNQGLSER²⁷⁶ | 13 |
| 12. | ³⁰²ESNPVTGNTCDNVKQRAALIDCLA³²⁵ | 24 |
| 13. | ³³⁷IKDVVT³⁴² | 6 |

| **EF-Tu protein** | | |
|---|---|---|
| 14. | ⁴EKYDRSK¹⁰ | 7 |
| 15. | ³⁷ARRLPTSVNQPKDYASIDAAPEERER⁶² | 26 |
| 16. | ¹⁴⁴VDDEELLE¹⁵² | 8 |
| 17. | ¹⁶⁵DFPGD¹⁶⁹ | 5 |
| 18. | ¹⁸¹ALEGDSKYEDI¹⁹¹ | 11 |
| 19. | ²⁰¹EYIPEPERDT²¹⁰ | 10 |
| 20. | ²⁶⁴EMFRKQLDEGL²⁷⁴ | 11 |
| 21. | ²⁸⁶VQRDEIE²⁹² | 7 |
| 22. | ³⁰⁰PGSINPHTKFKG³¹¹ | 12 |
| 23. | ³¹⁷SKDEGGRHTPFFNN³³⁰ | 14 |
| 24. | ³⁵⁰AGTEMVM³⁵⁶ | 7 |

| **ADA protein** | | |
|---|---|---|
| 25. | ³⁶IILPSSDKELRKYVIAPAQTESLVD⁶⁰ | 25 |
| 26. | ¹⁰⁵LSMDKGLTA¹¹³ | 9 |
| 27. | ¹⁴⁵SSHKTTK¹⁵¹ | 7 |
| 28. | ¹⁷⁴EFSYPTDS¹⁸¹ | 8 |
| 29. | ²²⁷TGQRD²³¹ | 5 |
| 30. | ²⁵³TKAASSIQSF²⁶² | 10 |
| 31. | ³⁰¹TKIE³⁰⁴ | 4 |
| 32. | ³¹⁹TSDSEKDTLLHKLQENYD³³⁶ | 18 |

| **FnBPA protein** | | |
|---|---|---|
| 33. | ¹⁹⁵TDVTSKVTVE²⁰⁴ | 10 |
| 34. | ³⁶⁸IKPNNGKTTSVTV³⁸⁰ | 13 |
| 35. | ²³²KFENGLHQGDYFDFT²⁶⁴ | 15 |
| 36. | ³⁴²GNYYAN³⁴⁷ | 6 |
| 37. | ⁴¹⁵ANTTDTSKFKEVTSNMSGNLNLQNNG⁴⁴⁰ | 26 |
| 38. | ⁴⁴³SLNIENLDKTYV⁴⁵⁴ | 12 |
| 39. | ³⁸⁸SNQNGNQPKVRIFEYLGNNEDIA⁴¹⁰ | 23 |
| 40. | ²⁷⁶EVLEGGKIR YTFTNDIEDKVDV²⁹⁷ | 22 |
| 41. | ³¹²VQTNGNQTITSTLNEEQTSKELD³³⁴ | 23 |
| 42. | ¹⁹⁵TDVTSKVT²⁰² | 8 |
| 43. | ²³²KFENGLHQGDYFDFT²⁴⁶ | 15 |
| 44. | ⁴¹⁵ANTTDTSKFKEVTSNMNLNLQNNGSYSLNIENLDKTYV⁴⁵⁴ | 38 |
| 45. | ³⁶⁸IKPNNGKTTSVTVT³⁸¹ | 14 |
| 46. | ⁴⁷⁴MVGHPYT⁴⁹¹ | 7 |
| 47. | ³⁸⁷GSNQNGNQPKVRIFEYLGNNEDIA⁴¹⁰ | 24 |
| 48. | ²⁶⁷NGSVVMATGEVLEGGKIRYTFTNDIEDKVDV²⁹⁷ | 31 |
| 49. | ³⁴²GNYYA³⁴⁶ | 5 |
| 50. | ⁴⁵⁸DGEYLNGT⁴⁶⁵ | 8 |
| 51. | ³⁵⁷KANNR³⁶¹ | 5 |

### Literature:

1. Hogeveen, H., Huijps, K., and Lam, T. J. G. M. (2011). Economic aspects of mastitis: New developments. N. Z. Vet. J. 59:16-23. doi: 10.1080/00480169.2011.547165.
2. Philpot, W. N., and Nickerson, S. C. (1991). Mastitis: Counter Attack: A Strategy to Combat Mastitis. Chicago.
3. He, W., Ma, S., Lei, L., He, J., Li, X., Tao, J., et al. (2020). Prevalence, etiology, and economic impact of clinical mastitis on large dairy farms in China. Vet. Microbiol. 242:108570. doi: 10.1016/J.VETMIC.2019.108570.
4. Dufour, S., Labrie, J., and Jacques, M. (2019). The Mastitis Pathogens Culture Collection. Microbiol. Resour. Announc. 8:133-52. doi: 10.1128/MRA.00133-19.
5. Lassa, H., Kubiak, J., and Makiska-Horodyska, M. (2013). Antibiotic susceptibility of the bacteria most often isolated from clinical mastitis in cows. Zycie Weterynaryjne. 88:651.
6. Wilson, D. J., Gonzalez, R. N., and Das, H. H. (1997). Bovine Mastitis Pathogens in New York and Pennsylvania: Prevalence and Effects on Somatic Cell Count and Milk Production. J. Dairy. Sci. 80:2592-8. doi: 10.3168/JDS.S0022-0302(97)76215-5.
7. Kulkarni, A. G., and Kaliwal, B. B. (2013). Bovine mastitis: a review. International Journal of Recent Scientific Research 4:543-8.
8. Yalçin, C. (2000). Cost of Mastitis in Scottish Dairy Herds with Low and High Subclinical Mastitis Problems. Turkish Journal of Veterinary and Animal Sciences. 24:465-72.
9. Park, H. M., Almeida, R. A., and Oliver, S. P. (2002). Identification of lactoferrin-binding proteins in Streptococcus dysgalactiae subsp. dysgalactiae and Streptococcus agalactiae isolated from cows with mastitis. FEMS. Microbiol. Lett. 207:87-90. doi: 10.1111/j.1574-6968.2002.tb11033.x.
10. Fang, W., and Oliver, S. P. (1999). Identification of lactoferrin-binding proteins in bovine mastitis-causing Streptococcus uberis. FEMS. Microbiol. Lett. 176:91-6. doi: 10.1111/j.1574-6968.1999.tb13647.x.
11. Laemmli, U. K. (1970). Cleavage of structural proteins during the assembly of the head of bacteriophage T4. Nature. 227:680-5. doi: 10.1038/227680a0.
12. Tristan A, Ying L, Bes M, Etienne J, Vandenesch F, Lina G. (2003). Use of multiplex PCR to identify Staphylococcus aureus adhesins involved in human hematogenous infections. J Clin Microbiol. 41:4465-7. doi: 10.1128/JCM.41.9.4465-4467.2003.
13. Banner M. A., Cunniffe J. G., Macintosh R. L., Foster T. J., Rohde H., Mack D., et al. (2007). Localized tufts of fibrils on Staphylococcus epidermidis NCTC 11047 are comprised of the accumulation-associated protein. J Bacteriol. 189:2793-804. doi: 10.1128/JB.00952-06
14. Galfrè G., Milstein C. (1981). Preparation of monoclonal antibodies: strategies and procedures. Methods Enzymol. 73:3-46. doi: 10.1016/0076-6879(81)73054-4.

## Claims

1. A method for detecting mastitis caused by *E. coli, S. uberis, S. agalactiae* or *S. aureus,* **characterized in that** the following antigens are tested in a biological specimen, in particular milk, from the udder of a tested cow:
- ompA protein from *E. coli* having amino acid sequence SEQ ID NO. 1 or epitopes contained therein, listed in Table 1 having an amino acid sequence selected from SEQ ID NO. 5-13,
- EF-Tu protein from *S. uberis* having amino acid sequence SEQ ID NO. 2 or epitopes contained therein, listed in Table 1 having an amino acid sequence selected from SEQ ID NO. 14-24,
- ADA protein from *S. agalactiae* having amino acid sequence SEQ ID NO. 3 or epitopes contained therein, listed in Table 1 having an amino acid sequence selected from SEQ ID NO. 25-32 and
- FnBPA protein from *S. aureus* having amino acid sequence SEQ ID NO. 4 or epitopes contained therein, listed in Table 1 having an amino acid sequence selected from SEQ ID NO. 33-51,
wherein:
- confirmed presence of the antigen from *E. coli* indicates that the udder of the cow tested is infected by *E. coli* causing mastitis,
- confirmed presence of the antigen from *S. uberis* indicates that the udder of the cow tested is infected by *S. uberis* causing mastitis,
- confirmed presence of the antigen from *S. agalactiae* indicates that the udder of the cow tested is infected by *S. agalactiae* causing mastitis, while
- confirmed presence of the antigen from *S. aureus* indicates that the udder of the cow tested is infected by *S. aureus* causing mastitis,

2. The method of claim 1, **characterized in that** for testing the presence of the antigens, antibodies specific for the antigens are used.

3. The method of claim 1, **characterized in that** the presence of the antigens is detected using an immunochromatographic test.

4. A diagnostic composition for detecting mastitis caused by *E. coli, S. uberis, S. agalactiae* or *S. aureus,* **characterized in that** it contains reagents, in particular antibodies, useful for detecting the following antigens:
- ompA protein from *E. coli* having amino acid sequence SEQ ID NO. 1 or epitopes contained therein, listed in Table 1 having an amino acid sequence selected from SEQ ID NO. 5-13,
- EF-Tu protein from *S. uberis* having amino acid sequence SEQ ID NO. 2 or epitopes contained therein, listed in Table 1 having an amino acid sequence selected from SEQ ID NO. 14-24,
- ADA protein from *S. agalactiae* having amino acid sequence SEQ ID NO. 3 or epitopes contained therein, listed in Table 1 having an amino acid sequence selected from SEQ ID NO. 25-32 and
- FnBPA protein from *S. aureus* having amino acid sequence SEQ ID NO. 4 or epitopes contained therein, listed in Table 1 having an amino acid sequence selected from SEQ ID No. 33-51.

5. The diagnostic composition of claim 4, **characterized in that** it contains monoclonal or polyclonal antibodies specific for the antigens of claim 4.

6. The diagnostic composition of claim 4, **characterized in that** it contains labelled antibodies.

## Patentansprüche

1. Verfahren zum Nachweis von Mastitis, die verursacht wird durch *E. coli, S. uberis, S. agalactiae* oder *S*. *aureus,* **dadurch gekennzeichnet, dass** die folgenden Antigene in einer biologischen Probe, insbesondere Milch, aus dem Euter einer getesteten Kuh untersucht werden:
- OmpA-Protein aus *E. coli* aufweisend die Aminosäuresequenz SEQ **ID** NO. 1 oder darin enthaltene Epitopen, aufgeführt in Tabelle 1, aufweisend eine Aminosäuresequenz, ausgewählt aus SEQ ID NO. 5-13,
- EF-Tu-Protein aus *S*. *uberis* aufweisend die Aminosäuresequenz SEQ ID NO. 2 oder darin enthaltene Epitopen, aufgeführt in Tabelle 1, aufweisend eine Aminosäuresequenz, ausgewählt aus SEQ ID NO. 14-24,
- ADA-Protein aus *S*. *agalactiae* aufweisend die Aminosäuresequenz SEQ ID NO. 3 oder darin enthaltene Epitopen, aufgeführt in Tabelle 1, aufweisend eine Aminosäuresequenz, ausgewählt aus SEQ ID NO. 25-32 und
- FnBPA-Protein aus *S*. *aureus* aufweisend die Aminosäuresequenz SEQ ID NO. 4 oder darin enthaltene Epitopen, aufgeführt in Tabelle 1, aufweisend eine Aminosäuresequenz, ausgewählt aus SEQ ID NO. 33-51,
wobei:
- das bestätigte Vorhandensein des Antigens von *E. coli* darauf hinweist, dass das Euter der getesteten Kuh mit *E. coli* infiziert ist, was zu Mastitis führt,
- das bestätigte Vorhandensein des Antigens von *S. uberis* darauf hinweist, dass das Euter der getesteten Kuh mit *S*. *uberis* infiziert ist, was zu Mastitis führt,
- das bestätigte Vorhandensein des Antigens von *S. agalactiae* darauf hinweist, dass das Euter der getesteten Kuh mit *S*. *agalactiae* infiziert ist, was Mastitis verursacht, während
- das bestätigte Vorhandensein des Antigens von *S*. aureus darauf hinweist, dass das Euter der getesteten Kuh mit *S*. *aureus* infiziert ist, was zu Mastitis führt,

2. Verfahren nach Anspruch 1, **dadurch gekennzeichnet, dass** zum Testen des Vorhandenseins der Antigene für die Antigene spezifische Antikörper verwendet werden.

3. Verfahren nach Anspruch 1, **dadurch gekennzeichnet, dass** das Vorhandensein der Antigene unter Verwendung eines immunochromatographischen Tests nachgewiesen wird.

4. Diagnostische Zusammensetzung zum Nachweis von Mastitis, verursacht durch *E*. *coli, S.* uberis, *S*. *agalactiae* oder *S*. *aureus,* **dadurch gekennzeichnet, dass** sie Reagenzien, insbesondere Antikörper, enthält, die zum Nachweis der folgenden Antigene geeignet sind:
- OmpA-Protein aus *E. coli* aufweisend die Aminosäuresequenz SEQ ID NO. 1 oder darin enthaltene Epitopen, aufgeführt in Tabelle 1, aufweisend eine Aminosäuresequenz, ausgewählt aus SEQ ID NO. 5-13,
- EF-Tu-Protein aus *S*. *uberis* aufweisend die Aminosäuresequenz SEQ ID NO. 2 oder darin enthaltene Epitopen, aufgeführt in Tabelle 1, aufweisend eine Aminosäuresequenz, ausgewählt aus SEQ ID NO. 14-24,
- ADA-Protein aus *S*. *agalactiae* aufweisend die Aminosäuresequenz SEQ **ID** NO. 3 oder darin enthaltene Epitopen, aufgeführt in Tabelle 1, aufweisend eine Aminosäuresequenz, ausgewählt aus SEQ ID NO. 25-32 und
- FnBPA-Protein aus *S*. *aureus* aufweisend die Aminosäuresequenz SEQ **ID** NO. 4 oder darin enthaltenen Epitope, aufgeführt in Tabelle 1 aufweisend eine Aminosäuresequenz, ausgewählt aus SEQ ID Nr. 33-51.

5. Diagnostische Zusammensetzung von Anspruch 4, **dadurch gekennzeichnet, dass** sie monoklonale oder polyklonale Antikörper enthält, die für die Antigene von Anspruch 4 spezifisch sind.

6. Diagnostische Zusammensetzung von Anspruch 4, **dadurch gekennzeichnet, dass** sie markierte Antikörper enthält.

## Revendications

1. Procédé pour détecter une mammite causée par *E. coli, S. uberis, S. agalactiae* ou *S. aureus,* **caractérisé en ce que** les antigènes suivants sont testés dans un spécimen biologique, en particulier le lait, provenant de la mamelle d'une vache testée :
- la protéine ompA de *E. coli* présentant la séquence d'acides aminés SEQ ID NO. 1 ou des épitopes qui y sont contenus, énumérés dans le tableau 1 présentant une séquence d'acides aminés choisie parmi SEQ ID NO. 5-13,
- la protéine EF-Tu de *S*. *uberis* présentant la séquence d'acides aminés SEQ ID NO. 2 ou des épitopes qui y sont contenus, énumérés dans le tableau 1 présentant une séquence d'acides aminés choisie parmi SEQ ID NO. 14-24,
- la protéine ADA de *S*. *agalactiae* présentant la séquence d'acides aminés SEQ ID NO. 3 ou des épitopes qui y sont contenus, énumérés dans le tableau 1 présentant une séquence d'acides aminés choisie parmi SEQ ID NO. 25-32 et
- la protéine FnBPA de *S*. *aureus* présentant la séquence d'acides aminés SEQ ID NO. 4 ou des épitopes qui y sont contenus, énumérés dans le tableau 1 présentant une séquence d'acides aminés choisie parmi SEQ ID NO. 33-51,
dans lequel :
- la présence confirmée de l'antigène de *E. coli* indique que la mamelle de la vache testée est infectée par *E. coli* causant une mammite,
- la présence confirmée de l'antigène de *S*. *uberis* indique que la mamelle de la vache testée est infectée par *S*. *uberis* causant une mammite,
- la présence confirmée de l'antigène de *S*. *agalactiae* indique que la mamelle de la vache testée est infectée par *S*. *agalactiae* causant une mammite, tandis que
- la présence confirmée de l'antigène de *S*. *aureus* indique que la mamelle de la vache testée est infectée par *S*. *aureus* causant une mammite,

2. Procédé selon la revendication 1, **caractérisé en ce que** pour tester la présence des antigènes, des anticorps spécifiques aux antigènes sont utilisés.

3. Procédé selon la revendication 1, **caractérisé en ce que** la présence des antigènes est détectée à l'aide d'un test immunochromatographique.

4. Composition diagnostique pour détecter une mammite causée par *E. coli, S. uberis, S*. *agalactiae* ou *S*. *aureus,* **caractérisée en ce qu'**elle contient des réactifs, en particulier des anticorps, utiles pour détecter les antigènes suivants :
- la protéine ompA de *E. coli* présentant la séquence d'acides aminés SEQ ID NO. 1 ou des épitopes qui y sont contenus, énumérés dans le tableau 1 présentant une séquence d'acides aminés choisie parmi SEQ ID NO. 5-13,
- la protéine EF-Tu de *S*. *uberis* présentant la séquence d'acides aminés SEQ ID NO. 2 ou des épitopes qui y sont contenus, énumérés dans le tableau 1 présentant une séquence d'acides aminés choisie parmi SEQ ID NO. 14-24,
- la protéine ADA de *S*. *agalactiae* présentant la séquence d'acides aminés SEQ ID NO. 3 ou des épitopes qui y sont contenus, énumérés dans le tableau 1 présentant une séquence d'acides aminés choisie parmi SEQ ID NO. 25-32 et
- la protéine FnBPA de *S*. *aureus* présentant la séquence d'acides aminés SEQ ID NO. 4 ou les épitopes qui y sont contenus, énumérés dans le tableau 1 présentant une séquence d'acides aminés choisie parmi SEQ ID No. 33-51.

5. Composition diagnostique selon la revendication 4, **caractérisée en ce qu'**elle contient des anticorps monoclonaux ou polyclonaux spécifiques aux antigènes selon la revendication 4.

6. Composition diagnostique selon la revendication 4, **caractérisée en ce qu'**elle contient des anticorps marqués.
